# EUROPEAN PATENT SPECIFICATION

(11) **EP 1 719 465 B1**
(45) Date of publication and mention of the grant of the patent: **12.03.2008**
(21) Application number: 06111602.6
(22) Date of filing: 23.03.2006
(51) Int. Cl.: A61B 17/17, A61B 19/00

(54) **Device for positioning the fixing screws of an intramedullary nail, particularly the distal screws**
Vorrichtung zum Anbringen von Distale Verriegelungsschrauben in intramedulären Nägeln
Dispositif de positionnement de vis de verrouillage distal pour un clou intramédullaire

(30) Priority: 02.05.2005 IT BO20050303
(43) Date of publication of application: 08.11.2006
(73) Proprietor: CITIEFFE S.r.l., 40012 Calderara di Reno (Bologna) (IT)
(72) Inventor: Mingozzi, Franco, 40012, CALDERARA DI RENO BO (IT); Dovesi, Alan, 40100, BOLOGNA (IT); Scoccianti, Alberto, 40012, LIPPO DI CALDERARA DI RENO BO (IT)
(74) Representative: Modiano, Micaela Nadia

(56) References cited:
- EP-A- 0 354 395
- WO-A-03/065907
- DE-U1- 29 815 700
- FR-A- 2 587 196

## Description

The present invention relates to a device for positioning the fixing screws of an intramedullary nail, particularly the distal screws.

It is known to treat the fractures of long bones (femur, tibia, humerus, radius and ulna) by using intramedullary nails, which are inserted in the medullary canal and are fixed at their opposite ends by means of through screws. While the fixing of the so-called proximal screws to the nail insertion end does not entail particular problems, the fixing of the so-called distal screws at the opposite end is often critical, owing to the fact that the nail, during insertion, undergoes torsions and deformations which produce a misalignment of the position of the screw passage holes which cannot be ascertained from the outside.

In order to ensure correct positioning of the fixing screws, devices are already known which allow to determine the position of the screw passage holes after the intramedullary nail has been inserted in the bone.

Devices of this kind are known, for example, from US-4,622,959, US-4,667,664 and US-6,702,823, from French Patents No. 2,705,558 and 2,715,615, and from EP-0493570 and EP-1358852.

Known devices, however, suffer from drawbacks, especially in relation to the fact that they do not allow to perform rapid positioning of the fixing screws, with the consequence of increasing the duration of surgical procedures for setting fractures and the exposure of the surgeon and of the patient to the radiation of exploratory X-rays.

DE 298 15 700 discloses an intramedullary nail fixing screw positioning device having a combination of features as set forth in the precharacterizing portion of the appended claim 1.

The aim of the present invention is to provide a device which allows to identify rapidly the precise placement of the screws, particularly the distal screws, so as to obviate the cited drawbacks.

In accordance with the invention, there is provided a device for positioning the fixing screws of an intramedullary nail inserted in the medullary canal of a long bone, particularly the distal screws, as defined in the appended claims.

Further characteristics and advantages of the present invention will become better apparent from the following detailed description of a preferred but not exclusive embodiment thereof, illustrated by way of non-limiting example in the accompanying drawings, wherein:
Figure 1 is a side view of the device and of the intramedullary nail associated therewith;
Figures 2 and 3 are views, taken from opposite sides, of the device of Figure 1;
Figure 4 is a perspective view of the device of Figure 1;
Figures 5 and 6 are views of the device in two different situations, in which the guiding holes of the alignment fixture are off-centered with respect to the distal holes;
Figure 7 is a view of the device in the position in which the guiding holes are perfectly aligned with the distal holes;
Figure 8 is a sectional view of the means designed for the transverse positioning of the alignment fixture.

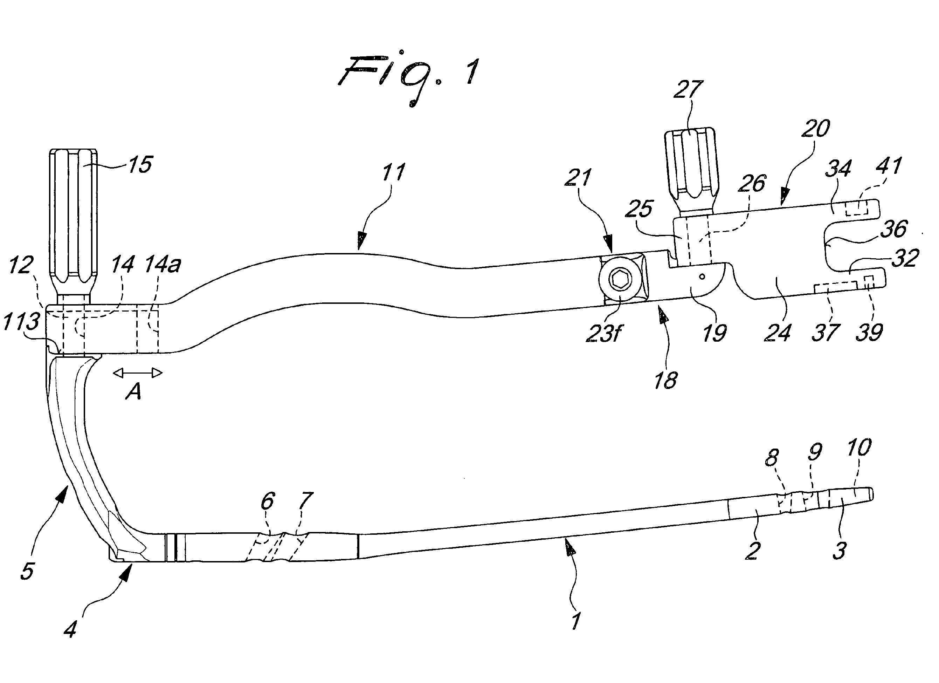

With reference to Figures 1, 2, 3 and 4, the reference numeral 1 designates a generic intramedullary nail provided with an end portion 2, which ends with a tip 3, by means of which it is inserted in the medullary canal of a long bone (femur, tibia, et cetera).

The end of the nail 1 which lies opposite the tip 3 is provided with securement means 4, by means of which the nail is fixed both axially and rotationally to an arm 5.

For the sake of brevity in description, the securement means are not described in detail, since they are fully conventional and unrelated to the subj ect of the present invention.

The nail 1 is slightly curved, so as to follow the shape of the medullary canal in which it is to be inserted, and two holes 6, 7 and 8, 9 are provided in each of its opposite ends for the passage of the proximal and distal screws, respectively, by means of which the nail is fixed in the medullary canal. In particular, the two holes 8, 9 are formed in the end portion 2 and are termed distal, while the holes 6, 7 are termed proximal.

Advantageously, the hole 9 is elongated axially, and the tip 3, for reasons which will become better apparent hereinafter, is provided with an axial slot 10 which is coplanar with respect to the hole 9.

The arm 5 has a curved shape in order to connect to the end of a bar 11 provided with weight reduction openings 11 a, which lies substantially parallel to the nail in the direction A and externally with respect to the bone in which the nail must be inserted and with respect to the muscle tissues that cover said bone.

The connection of the arm 5 to the bar 11 is provided by means of a clamp, which is constituted by a threaded pivot 12, which protrudes from a flat region 13 of the arm 5 through a hole 14 of the bar, and by a securement knob 15, which is screwed onto the pivot 12. As an alternative, the pivot 12 can be driven through a second hole 14a of the bar if the device requires adjustments when shorter nails have to be used.

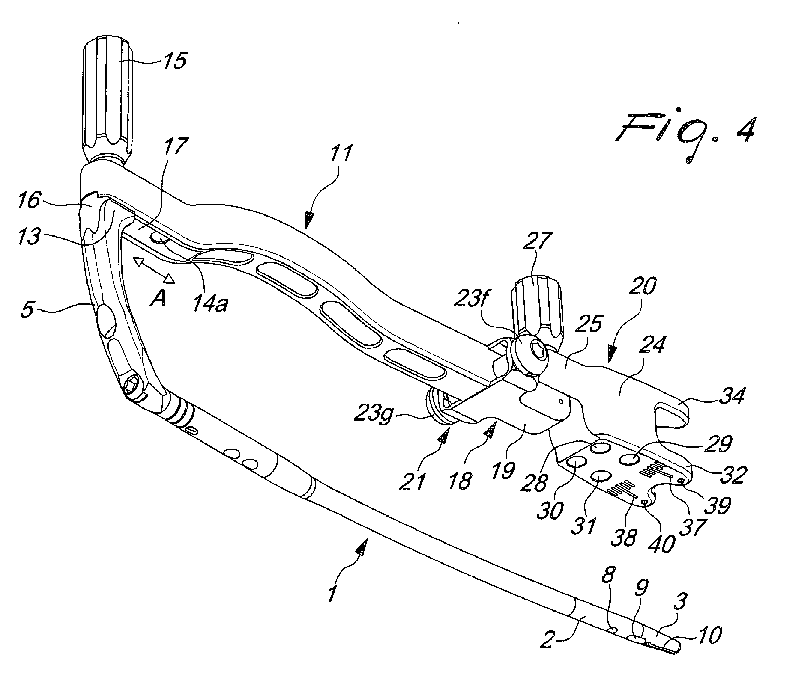

In order to allow the bar 11 a firm positioning with respect to the arm 5 and therefore with respect to the nail, a side-fit coupling is provided which is composed of a tenon 16 (Figure 4) which is formed on the flat portion 13 of the arm 5 and is engaged in a mortise 17, which protrudes in the direction A and is formed in the face of the bar 11 which lies opposite the flat region 13.

A support 18 is fitted at the end of the bar 11 which lies opposite the knob 15 and protrudes so as to form a bracket 19 on which an alignment or centering fixture, generally designated by the reference numeral 20, is arranged.

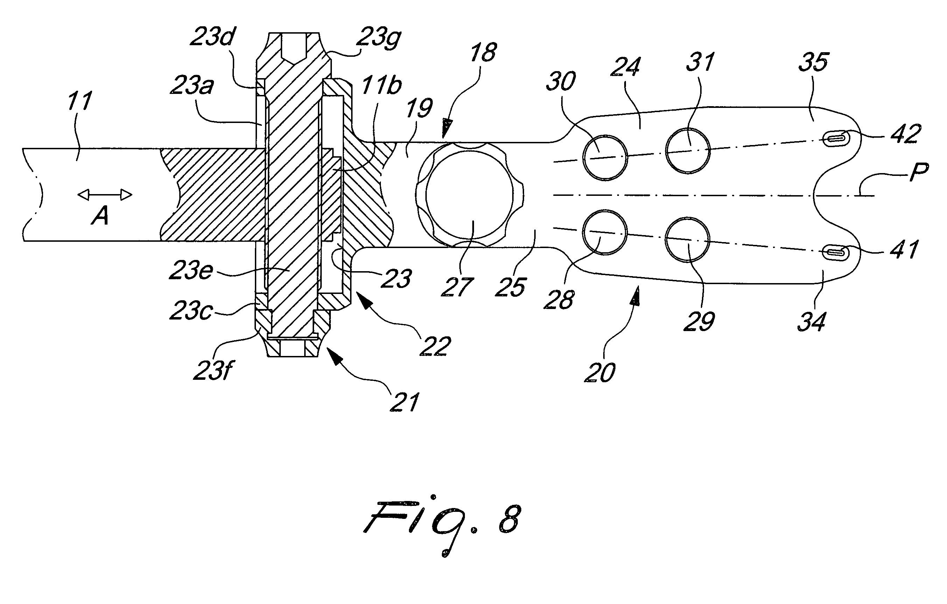

The support 18 is guided on the bar 11 so that it can perform a translational motion at right angles to the direction A. For this purpose (see in particular Figure 8), there is a screw-type actuation element, hereinafter referenced as slider 21 for convenience in description, which is composed of a recess 22 which is formed in the support 18 and has a substantially rectangular prism-like cavity 23, which is formed by two walls 23a, 23b, which are mutually opposite and arranged transversely to the bar 11, and by two walls 23c, 23d, which connect the walls 23a, 23b at the ends.

The cavity 23 is open toward the bar 11, which engages therein with an end portion 11b, which is kept guided between the walls 23a, 23b. In this end portion 11b there is a threaded hole, which is perpendicular to the direction A and in which a screw 23e is screwed, said screw being able to rotate in the walls 23c, 23d. The opposite ends of the screw 23e protrude from the recess 22 through the walls 23c, 23d and are provided with respective rings 23f, 23g, which when actuated allow to adjust the position of the support 18 to the right or to the left with respect to the bars 1.

The alignment fixture 20 comprises a head 24 made of a material which is transparent to radiation and consists of a substantially prism-like block, from which an eyelet 25 protrudes; the head is fixed to the bracket 19 by means of said eyelet by using a clamp which is composed of a threaded pivot 26 (Figure 1), which protrudes upward from the bracket 19, and by a knob 27, which is screwed on the pivot 26 in order to secure the eyelet on the bracket 19.

Two pairs of holes 28, 29 and 30, 31 are formed in the head 24 and are referenced hereinafter as guiding holes in relation to their function of guiding the distal screws.

The guiding holes 28, 29 and 30, 31 are arranged mirror-symmetrically with respect to a central plane P (Figure 8) of the head 24 and of the bar 11. More precisely, the pairs of holes 28, 29 and 30, 31 are arranged along lines which, as will become better apparent from Figure 8, diverge slightly with respect to the plane P and enclose, with the central plane P, an angle which is substantially equal to the deviation angle of the end portion 2 of the nail 1 with respect to the plane P. Further, the distance between the holes 28, 29 and 30, 31 is equal to the distance between the distal holes 8, 9 of the nail, so that before the nail is installed in the bone it is possible to check the alignment status of the guiding holes 28, 29 and 30, 31 with respect to the distal holes 8, 9 of the nail 1.

In practice, the choice of the pair of holes 28, 29 or 30, 31 with which the holes 8, 9 are to be aligned depends, as is evident, on the direction in which the deviation of the nail must be oriented, depending on whether the nail is to be implanted in the right or left bone.

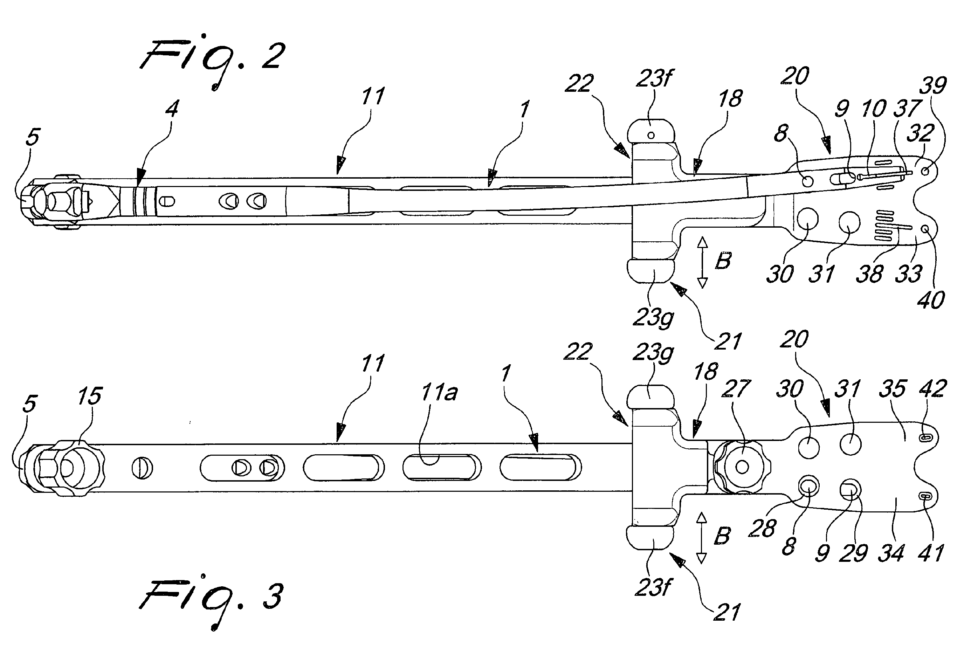

As shown more clearly by Figures 2 and 3, two expansions 32, 33 and respectively 34, 35 protrude from the head 24, on the opposite side with respect to the eyelet 25, and are mutually superimposed so as to form between them a separation cavity 36.

Proximate to the expansions 32, 33 which face the nail 1 there are respective divisions, constituted by mutually parallel bars, the central ones 37, 38 being longer than the others and being aligned with the respective pairs of guiding holes 28, 29 and 30, 31. Further, the central bars 37, 38 are directed toward a respective circular point, termed bore 39, 40 hereinafter for the sake of convenience, which is provided at the end of the expansions 32, 33.

The divisions, the bars 37, 38 and the bores 39, 40 are constituted conveniently by slots or hollows which are filled with a material which is opaque to radiation, so as to be visible during the X-rays which is taken by means of an image intensifier by the medical team performing the implantation of the intramedullary nail and which, as is known, are intended to check whether the alignment of the distal holes 8, 9 with the guiding holes 28, 29 or 30, 31 has been reached which allows to apply the distal screws by means of which the nail is secured within the medullary canal of the bone.

The fixture 20 is completed by two ovalized rings 41, 42, which are formed in the pair of expansions 34, 35 and are elongated in the same direction as the bars 37, 38. The rings 41, 42, like the bars 37, 38, are formed by recesses which are filled with a material which is opaque to radiation. The rings 41, 42 cooperate with the respective bores 39, 40, so as to establish a criterion of acceptability of the alignment of the guiding holes 28, 29 with the distal holes 8, 9.

The setting of the device according to the invention is described hereinafter, assuming hypothetically an initial situation (i.e., when the nail has not yet been implanted into the bone) in which the distal holes 8, 9 of the nail are aligned with the guiding holes 28, 29, as shown in Figure 2.

Assume now that the nail 1 has been implanted into the bone and that during insertion the nail has undergone a deformation along the transverse axis of the bar 11, causing the offset of the guiding holes 28, 29 with respect to the distal holes 8, 9.

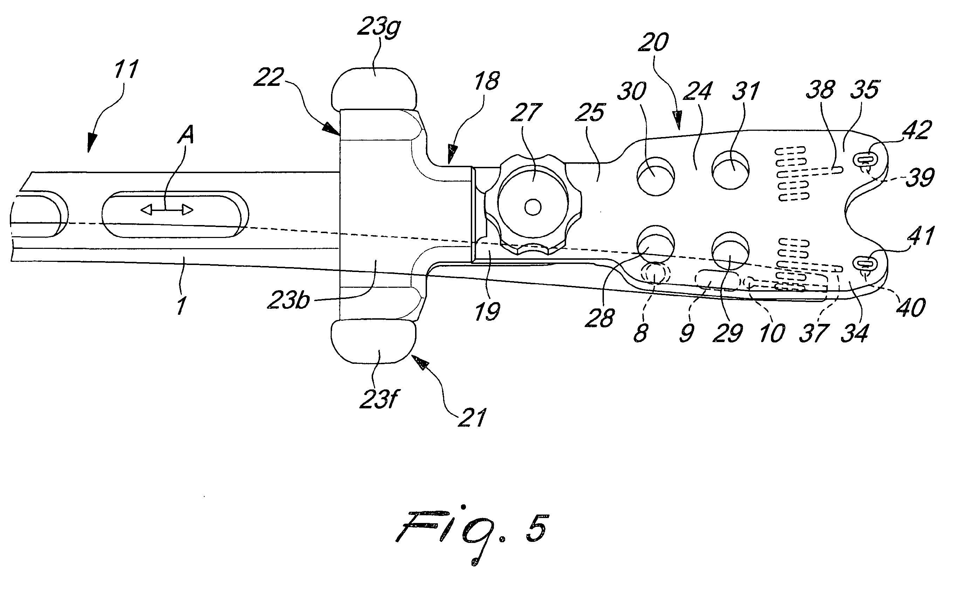

In a first step, the position of the image intensifier with respect to the plane of the alignment fixture 20 is checked. Evaluation of the extent of the flexing undergone by the nail can in fact be biased if the position of the image intensifier is not perpendicular to the plane of the alignment fixture (Figure 5). The correct position of the image intensifier is found when the bore 39 is arranged within the ring 41 (or when the bore 40 is arranged within the ring 42).

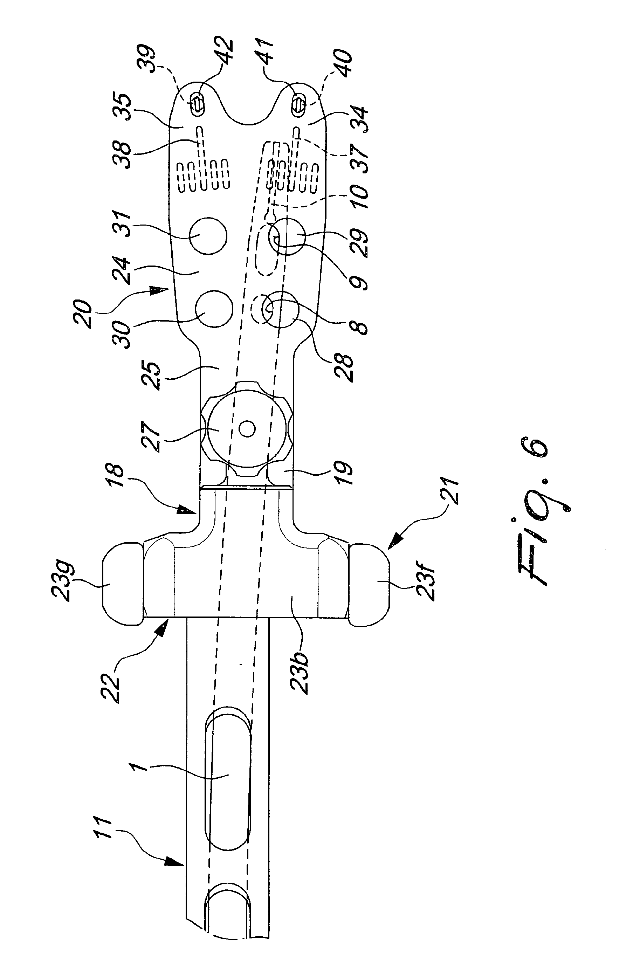

At this point, it is possible to determine the extent of the movement of the end portion 2 of the nail with respect to the bar 37 (or with respect to the bar 38) (Figure 6).

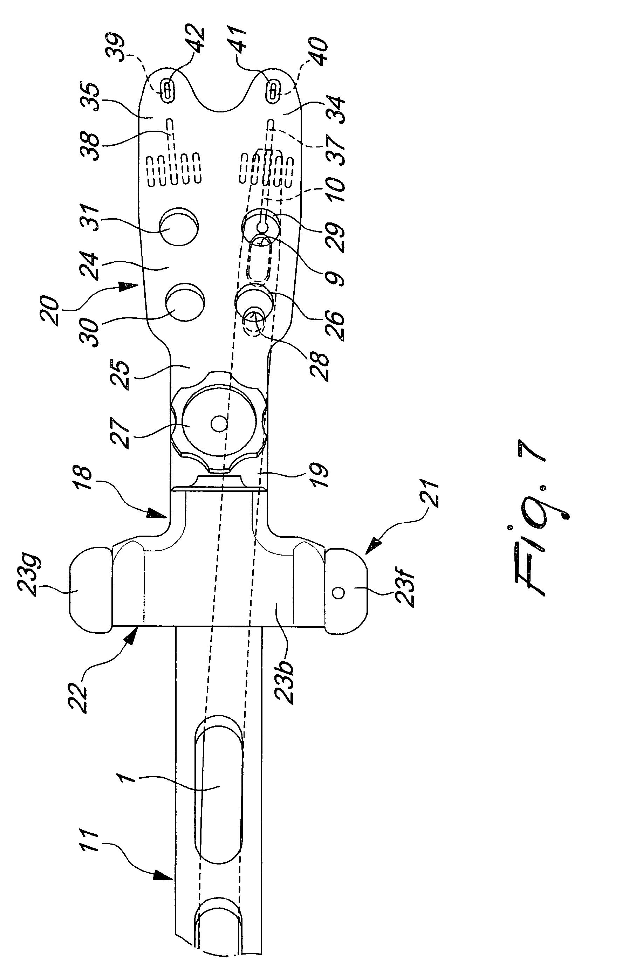

Alignment is established by acting on the slider 21 so as to move the head 24 by the same distance measured between the position of the slot 10 of the nail on the divisions arranged laterally with respect to the central bar 37 (or 38). This method ensures the coaxiality of the guiding holes 28, 29 with respect to the distal holes 8, 9, which allows the correct application of the distal screws (Figure 7).

As it is evident, the described invention perfectly achieves the intended aim and objects. Numerous modifications and variations are possible in the practical embodiment of the invention, and all are within the scope of the appended claims.

Thus, for example, instead of the two holes 14 and 14a it is possible to provide a single slot, which is elongated in the direction of the bar in order to allow continuous adjustment of the position of the arm along the bar.

One advantage of the device is its adaptability to nails of different lengths and shapes thanks to the possibility to vary the position of the arm 5 on the bar 11, by acting on the knob 15, and the orientation of the head 24 on the bracket 19, by acting on the knob 27.

Where technical features mentioned in any claim are followed by reference signs, those reference signs have been included for the sole purpose of increasing the intelligibility of the claims and accordingly such reference signs do not have any limiting effect on the interpretation of each element identified by way of example by such reference signs.

## Claims

1. A combination of an intramedullary nail (1) and a positioning device for positioning the fixing screws of said intramedullary nail (1) inserted in the medullary canal of a long bone, particularly the distal screws, and provided with distal holes (8, 9) for the passage of said screws, the positioning device comprising:
a bar (11),
an arm (5) which has a first end provided with means (4) for securing said intramedullary nail (1) and a second end connected to said bar (11), so that said bar lies substantially in the direction (A) of said nail (1),
an alignment fixture (20), which is mounted on said bar (11) at the opposite end with respect to the end for connection to said arm (5) and comprises a head (24) made of a material which is transparent to radiation and is provided with guiding holes (28-31) for said distal screws, said fixture (20) being arrangeable transversely to the axis of said nail (1) in order to bring said guiding holes (28-31) into alignment with said distal holes (8, 9),
**characterized in that** said head (24) comprises a division formed by bars filled with radio-opaque material, including a central bar (37) of which is aligned with a pair of guiding holes (28, 29) and can be aligned, by means of a translational motion of the alignment fixture (20), in a transverse direction (B) with respect to the intramedullary nail with a slot (10) which is formed axially at the end (3) of said nail (1) and is coplanar with respect to the distal holes (8, 9).

2. The combination according to claim 1, **characterized in that** said alignment fixture (20) comprises a support (18), which is guided on said bar so that it can perform a translational motion in a said transverse direction (B) with respect to said nail, said support (18) supporting said head (24) and a slider (21), which is adapted to adjust the transverse position of said head with respect to said nail.

3. The combination according to one of claims 1 and 2, **characterized in that** said head (24) comprises at least one pair of said pair of guiding holes (28, 29), which are arranged on a line which can be substantially parallel on the axis of the end portion (2) of the nail (1) and can be aligned with the distal holes (8, 9).

4. The combination according to one of claims 1 and 2, **characterized in that** said head (24) comprises two pairs of said pair of guiding holes (28-31), which are arranged mirror-symmetrically with respect to each other relative to a central plane (P) of the head, the guiding holes of each pair (28, 29 and 30, 31) being arranged along a line which can be substantially parallel along the axis of the end portion (2) of the nail (1) and can be aligned with the distal holes (8, 9).

5. The combination according to claim 4, **characterized in that** said head (24) comprises two divisions, each formed by said bars filled with radio-opaque material, a central one (37, 38) of which is aligned with a respective pair of guiding holes (28-31) and can be aligned, by means of the translational motion of the alignment fixture (20) in a transverse direction (B) with respect to the intramedullary nail (1), with a slot (10) which is formed axially at the end of said nail and is coplanar with respect to the distal holes (8, 9).

6. The combination according to claim 3, **characterized in that** said head (24) comprises superimposed expansions (32, 34), in one (34) of which there is an ovalized ring (41), which is aligned with said central bar (37) of the division, while in the other expansion (32) there is a bore (39) which can be aligned with said ring (41).

7. The combination according to claim 5, **characterized in that** said head (24) comprises two pairs of expansions (32-35), one pair (32, 33) being superimposed on the other one (34, 35), elongated rings (41, 42) being formed in one (32, 33) of said pairs and being aligned with the central bar (37, 38) of the respective divisions, respective bores (39, 40), which can be aligned with the respective rings (41, 42) being provided in the other pair (34, 35).

8. The combination according to claim 7, **characterized in that** said rings (41, 42) and said bars (37, 38) are formed by recesses of the head (24) and of the expansions (32-35), which are filled with a material which is opaque to X-rays.

9. The combination according to claim 2, **characterized in that** said slider (21) comprises a cavity (23), which is formed in said support (18) and in which an end portion (11b) of said bar (11) is guided, a screw (23e) being supported rotatably in said cavity, said screw (23e) being screwed through said end portion and being provided with rings (23f, 23g), in order to move said end portion (11b) within said cavity (23) and adjust the position of said support with respect to said bar (11).

10. The combination according to any one or more of the preceding claims, **characterized in that** said second end is provided with means (12-15) which connect said arm (5) to said bar (11) so that said arm (5) can be arranged along said bar (11) in the direction (A) of said nail (1).

## Patentansprüche

1. Eine Kombination eines intramedullären Nagels (1) und eine Positionierungsvorrichtung zum Positionieren der Befestigungsschrauben des intramedullären Nagels (1), der im medullären Kanal eines langen Knochens eingefügt ist, im Besonderen die distalen Schrauben, und mit distalen Löchern (8, 9) für den Durchgang der Schrauben bereitgestellt ist, die Positioniervorrichtung umfassend:
eine Schiene (11),
einen Arm (5), der ein erstes Ende aufweist, das mit Mitteln (4) zum Sichern des intramedullären Nagels (1) bereitgestellt ist, und ein zweites Ende, das mit der Schiene (11) verbunden ist, damit die Schiene im Wesentlichen in der Richtung (A) des Nagels (1) liegt,
eine Ausrichtungsvorrichtung (20), die an der Schiene (11) an dem in Bezug auf das Ende für die Verbindung an den Arm (5) gegenüberliegenden Ende angebracht ist und einen Kopf (24) umfasst, der aus einem Material besteht, das strahlungsdurchlässig ist und mit Führungslöchern (28-31) für die distalen Schrauben bereitgestellt ist, wobei die Vorrichtung (20) quer zur Achse des Nagels (1) anbringbar ist, um die Führungslöcher (28-31) in Ausrichtung mit den distalen Löchern (8, 9) zu bringen,
**dadurch gekennzeichnet, dass** der Kopf (24) eine Abteilung umfasst, der von mit röntgenstrahlenundurchlässigem Material gefüllten Schienen gebildet ist, einschließlich eine Zentralschiene (37), die mit einem Paar Führungslöchern (28, 29) ausgerichtet ist und mittels einer Translationsbewegung der Ausrichtungsvorrichtung (20) in einer Querrichtung (B) in Bezug auf den intramedullären Nagel mit einem Schlitz (10) ausrichtbar ist, der axial am Ende (3) des Nagels (1) ausgebildet ist und in Bezug auf die distalen Löcher (8, 9) koplanar ist.

2. Die Kombination gemäß Anspruch 1, **dadurch gekennzeichnet, dass** die Ausrichtungsvorrichtung (20) eine Halterung (18) umfasst, die auf der Schiene geführt ist, damit sie in Bezug auf den Nagel eine Translationsbewegung in der Querrichtung (B) ausführen kann, wobei die Halterung (18) den Kopf (24) und einen Schieber (21) stützt, der angepasst ist, die Querposition des Kopfes in Bezug auf den Nagel zu justieren.

3. Die Kombination gemäß einem der Ansprüche 1 und 2, **dadurch gekennzeichnet, dass** der Kopf (24) mindestens ein Paar des Paars von Führungslöchern (28, 29) umfasst, die auf einer Linie angeordnet sind, die im Wesentlichen parallel auf der Achse des Endbereichs (2) des Nagels (1) sein kann und mit den distalen Löchern (8, 9) ausrichtbar ist.

4. Die Kombination gemäß einem der Ansprüche 1 und 2, **dadurch gekennzeichnet, dass** der Kopf (24) zwei Paare des Paars von Führungslöchern (28-31) umfasst, die bezüglich einer zentralen Fläche (P) des Kopfs in Bezug aufeinander spiegelsymmetrisch angeordnet sind, wobei die Führungslöcher jedes Paares (28, 29 und 30, 31) entlang einer Linie angeordnet sind, die entlang der Achse des Endbereichs (2) des Nagels (1) im Wesentlichen parallel sein kann und mit den distalen Löchern (8, 9) ausrichtbar ist.

5. Die Kombination gemäß Anspruch 4, **dadurch gekennzeichnet, dass** der Kopf (24) zwei Abteilungen umfasst, jede davon von den mit röntgenstrahlenundurchlässigem Material gefüllten Schienen gebildet, eine Zentralschiene (37, 38), welche mit einem entsprechenden Paar von Führungslöchern (28-31) ausgerichtet ist und mittels der Translationsbewegung der Ausrichtungsvorrichtung (20) in einer in Bezug auf den intramedullären Nagel (1) Querrichtung (B) mit einem Schlitz (10) ausrichtbar ist, der axial am Ende des Nagels ausgebildet ist und in Bezug auf die distalen Löcher (8, 9) koplanar ist.

6. Die Kombination gemäß Anspruch 3, **dadurch gekennzeichnet, dass** der Kopf (24) übereinandergelagerte Expansionen (32, 34) umfasst, in einem (34) gibt es einen ovalisierten Ring (41), der mit der Zentralschiene (37) der Abteilung ausgerichtet ist, während es in der anderen Expansion (32) eine Bohrung (39) gibt, die mit dem Ring (41) ausrichtbar ist.

7. Die Kombination gemäß Anspruch 5, **dadurch gekennzeichnet, dass** der Kopf (24) zwei Paare von Expansionen (32-35) umfasst, wobei ein Paar (32, 33) dem anderen (34, 35) überlagert ist, wobei Längsringe (41, 42) in einem (32, 33) der Paare ausgebildet sind und mit der Zentralschiene (37, 38) der entsprechenden Abteilungen ausgerichtet sind, wobei entsprechende Bohrungen (39, 40), die mit den entsprechenden Ringen (41, 42) ausrichtbar sind, in dem anderen Paar (34, 35) bereitgestellt sind.

8. Die Kombination gemäß Anspruch 7, **dadurch gekennzeichnet, dass** die Ringe (41, 42) und die Schienen (37, 38) durch Aussparungen des Kopfes (24) und der Expansionen (32-35) gebildet sind, die mit einem Material gefüllt sind, das für Röntgenstrahlen undurchlässig ist.

9. Die Kombination gemäß Anspruch 2, **dadurch gekennzeichnet, dass** der Schieber (21) einen Hohlraum (23) umfasst, der in der Halterung (18) ausgebildet ist und in dem ein Endbereich (11b) der Schiene (11) geführt ist, wobei eine Schraube (23e) drehbar in dem Hohlraum gelagert ist, wobei die Schraube (23e) durch den Endbereich geschraubt ist und mit Ringen (23f, 23g) bereitgestellt ist, um den Endbereich (11b) innerhalb des Hohlraums (23) zu bewegen und die Position der Halterung in Bezug auf die Schiene (11) zu justieren.

10. Die Kombination gemäß irgendeinem der vorhergehenden Ansprüche, **dadurch gekennzeichnet, dass** das zweite Ende mit Mitteln (12-15) bereitgestellt ist, die den Arm (5) mit der Schiene (11) verbinden, damit der Arm (5) entlang der Schiene (11) in der Richtung (A) des Nagels (1) anbringbar ist.

## Revendications

1. Combinaison d'un clou intramédullaire (1) et d'un dispositif de positionnement pour positionner les vis de verrouillage dudit clou intramédullaire (1) inséré dans le canal médullaire d'un os long, en particulier les vis distales, et muni de trous distaux (8, 9) pour le passage desdites vis, le dispositif de positionnement comprenant:
une barre (11),
un bras (5) qui présente une première extrémité munie de moyens (4) pour assurer ledit clou intramédullaire (1) et une deuxième extrémité reliée à ladite barre (11), de sorte que la barre s'étende sensiblement dans la direction (A) dudit clou (1),
un appareil d'alignement (20) qui est monté sur ladite barre (11) à l'extrémité opposée par rapport à l'extrémité pour la connexion audit bras (5) et qui comprend une tête (24) faite en un matériau transparent aux radiations et qui est munie de trous de guidage (28-31) pour lesdites vis distales, ledit appareil (20) pouvant être agencé transversalement à l'axe dudit clou (1) afin d'amener en alignement lesdits trous de guidage (28-31) avec lesdits trous distaux (8, 9),
**caractérisée en ce que** ladite tête (24) comprend une division formée par des barres remplies de matériau opaque aux radiations, incluse une barre centrale (37) qui est alignée avec une paire de trous de guidage (28, 29) et qui peut être alignée, au moyen d'un déplacement translationnel de l'appareil d'alignement (20), dans une direction transversale (B) par rapport au clou intramédullaire, avec une fente (10) qui est formée axialement au niveau de l'extrémité (3) dudit clou (1) et qui est coplanaire par rapport aux trous distaux (8, 9).

2. Combinaison selon la revendication 1, **caractérisée en ce que** l'appareil d'alignement (20) comprend un support (18) qui est guidé sur ladite barre de sorte à pouvoir effectuer un déplacement translationnel dans ladite direction transversale (B) par rapport audit clou, ledit support (18) supportant ladite tête (24) et un coulisseau (21), qui est adapté pour ajuster la position transversale de ladite tête par rapport audit clou.

3. Combinaison selon l'une des revendications 1 et 2, **caractérisée en ce que** ladite tête (24) comprend au moins une paire desdites paires de trous de guidage (28, 29) qui sont agencés sur une ligne qui peut être sensiblement parallèle sur l'axe de la partie finale (2) du clou (1) et qui peuvent être alignés avec les trous distaux (8, 9).

4. Combinaison selon l'une des revendications 1 et 2, **caractérisée en ce que** ladite tête (24) comprend deux paires desdites paires de trous de guidage (28 à 31) qui sont agencés de façon spéculaire entre eux par rapport à un plan central (P) de la tête, les trous de guidage de chaque paire (28, 29 et 30, 31) étant agencés le long d'une ligne qui peut être sensiblement parallèle le long de l'axe de la partie finale (2) du clou (1) et pouvant etre alignés avec les trous distaux (8, 9).

5. Combinaison selon la revendication 4, **caractérisée en ce que** ladite tête (24) comprend deux divisions, chacune formée par ladite barres remplies de matériau opaque aux radiations, l'une desquelles centrale (37, 38) qui est alignée avec une paire respective de trous de guidage (28 à 31) et qui peut être alignée, au moyen du mouvement translationnel de l'appareil d'alignement (20) dans une direction transversale (B) par rapport au clou intramédullaire (1), avec une fente (10) qui est formée de façon axiale au niveau de l'extrémité dudit clou et qui est coplanaire par rapport aux trous distaux (8, 9).

6. Combinaison selon la revendication 3, **caractérisée en ce que** ladite tête (24) comprend des expansions superposées (32, 34), dans l'une (34) desquelles se trouve un anneau ovalisé (41), qui est aligné avec ladite barre centrale (37) de la division, alors que dans l'autre expansion (32) se trouve un rond (39) qui peut être aligné avec ledit anneau (41).

7. Combinaison selon la revendication 5, **caractérisée en ce que** ladite tête (24) comprend deux paires d'expansions (32 à 35), une paire (32, 33) étant superposée à l'autre (34, 35), des anneaux allongés (41, 42) étant formés dans l'une (32, 33) desdites paires et étant alignés avec la barre centrale (37, 38) des divisions respectives, des ronds respectifs (39, 40) qui peuvent être alignés avec les anneaux respectifs (41, 42) étant prévus dans l'autre paire (34, 35)

8. Combinaison selon la revendication 7, **caractérisée en ce que** lesdits anneaux (41, 42) et lesdites barres (37, 38) sont formés par des évidements de la tête (24) et des expansions (32 à 35) qui sont remplis d'un matériau qui est opaque aux rayons X.

9. Combinaison selon la revendication 2, **caractérisée en ce que** ledit coulisseau (21) comprend une cavité (23) qui est formée dans ledit support (18) et dans laquelle une partie finale (11b) de ladite barre (11) est guidée, une vis (23e) étant supportée de façon à pouvoir pivoter dans ladite cavité, ladite vis (23e) étant vissée à travers ladite partie finale et étant munie d'anneaux (23f, 23g) afin de déplacer ladite partie finale (11b) dans ladite cavité (23) et d'ajuster la position dudit support par rapport à ladite barre (11).

10. Combinaison selon l'une quelconque ou plusieurs des revendications précédentes, **caractérisée en ce que** ladite deuxième extrémité est munie de moyens (12 à 15) qui relient ledit bras (5) à ladite barre (11) de sorte que ledit bras (5) peut être agencé le long de ladite barre (11) dans la direction (A) dudit clou (1).
